# EUROPEAN PATENT SPECIFICATION

(11) **EP 3 409 272 B1**
(45) Date of publication and mention of the grant of the patent: **24.06.2020**
(21) Application number: 18169614.7
(22) Date of filing: 26.04.2018
(51) Int. Cl.: A61K 9/20, A61K 31/4152, A61P 7/02

(54) **PHARMACEUTICAL COMPOSITION COMPRISING ELTROMBOPAG OLAMINE, REDUCING SUGAR, AND POLYMERIC BINDER**
PHARMAZEUTISCHE ZUSAMMENSETZUNG MIT ELTROMBOPAG OLAMIN, REDUZIERENDEM ZUCKER UND POLYMEREM BINDEMITTEL
COMPOSITION PHARMACEUTIQUE COMPRENANT DE L'ELTROMBOPAG OLAMINE, DU SUCRE RÉDUCTEUR ET UN LIANT POLYMÉRIQUE

(30) Priority: 07.03.2018 IN 201811008417
(43) Date of publication of application: 05.12.2018
(73) Proprietor: Alfred E. Tiefenbacher (GmbH & Co. KG), 22767 Hamburg (DE)
(72) Inventor: STAVER, Ruslan, 22607 Hamburg (DE); PRATHAP, Vamshi Ramana, 502 319 Telangana (IN); VADLA, Hari Kiran Chary, 502 319 Telangana (IN); RALLABANDI, Bala Ramesha Chary, 502 319 Telangana (IN); SCHLEHAHN, Hendrik, 23843 Travenbrück (DE)
(74) Representative: Feldmann, Ute

(56) References cited:
- EP-A1- 1 207 155
- WO-A1-2012/121957
- WO-A2-03/098992
- US-A1- 2008 090 787
- NISHANT D. KAMBLI ET AL: "Synthesis and characterization of microcrystalline cellulose powder from corn husk fibres using bio-chemical route", CELLULOSE, vol. 24, no. 12, 12 October 2017 (2017-10-12), pages 5355-5369, XP055543193, Netherlands ISSN: 0969-0239, DOI: 10.1007/s10570-017-1522-4

## Description

### Technical Field:

The present invention relates to a pharmaceutical tablet composition comprising eltrombopag olamine, one or more reducing sugars, and one or more polymeric binder agents, a production process therefore, a pharmaceutical tablet composition comprising eltrombopag olamine, one or more reducing sugars, and one or more polymeric binder agents obtainable by the production process, a use / method of use of the pharmaceutical tablet compositions in the treatment or prophylaxis of immune (idiopathic) thrombocytopenic purpura (ITP), thrombocytopenia and/or acquired severe aplastic anaemia (SAA).

### Background of the invention:

3'-(N'-[1-(3,4-dimethylphenyl)-3-methyl-5-oxo-1,5-dihydropyrazol-4-ylidene]hydrazino}-2'-hydroxy-biphenyl-3-carboxylic acid - 2-aminoethanol (1:2) (hereinafter and in the context of the present invention called eltrombopag olamine, CAS 496775-62-3) is a non-peptide haematopoietic receptor agonist, which mimics haematopoietic growth factors, including thrombopoietin (TPO), particularly enhancing platelet production and, thus, is particularly useful in the treatment of thrombocytopenia (see e.g. WO 03/098992 A2 and WO 2008/136843A1).

In Europe eltrombopag olamine is approved under the tradename Revolade® comprising eltrombopag olamine in an amount corresponding to 12.5 mg, 25 mg, 50 mg, and 75 mg eltrombopag free acid in tablet form. In USA eltrombopag olamine is approved under the tradename Promacta® comprising eltrombopag olamine in an amount corresponding to 12.5 mg, 25 mg, 50 mg, 75 mg and 100 mg eltrombopag free acid in tablet form. Revolade® tablets are packed in Alu-Alu blisters, whereas Promacta® tablets are packed in HDPE bottles.

In Europe, Revolade® is indicated for adult chronic immune (idiopathic) thrombocytopenic purpura (ITP) splenectomised patients who are refractory to other treatments (e.g. corticosteroids, immunoglobulins). Revolade® may be considered as second line treatment for adult non-splenectomised patients where surgery is contraindicated.

Revolade® is also indicated in adult patients with chronic hepatitis C virus (HCV) infection for the treatment of thrombocytopenia, where the degree of thrombocytopenia is the main factor preventing the initiation or limiting the ability to maintain optimal interferon-based therapy.

Revolade® is furthermore indicated in adult patients with acquired severe aplastic anaemia (SAA) who were either refractory to prior immunosuppressive therapy or heavily pretreated and are unsuitable for haematopoietic stem cell transplantation.

In WO 03/098992 A2 an eltrombopag tablet composition is disclosed in example 6 which comprises in addition to 8.45 mg eltrombopag olamine, furthermore 112 mg microcrystalline cellulose, 70 mg lactose, 8 mg sodium starch glycolate and 2 mg magnesium stearate.

It is, however, known, that eltrombopag olamine undergoes a Maillard reaction with respective pharmaceutically acceptable excipients, such as reducing sugars, e.g. lactose. It is known that eltrombopag olamine is degraded in presence of lactose and forms impurities, which can be measured in the pharmaceutical tablet composition. Mr. Kapsi, one of the inventors of WO 2008/136843 A1 provided in the corresponding US examination of US 2010/0040684 A1 a declaration (in the context of the present application called "Kapsi declaration") disclosing experimental stability data for the use of the lactose containing eltrombopag tablet composition of example 6 in WO 03/098992 A2 in comparison to a tablet formulation, which is free of reducing sugars using mannitol instead.

According to the Kapsi declaration, the eltrombopag olamine tablet formulation comprising lactose shows an *"increase in degradation products when compared to the mannitol based formulation (4 fold difference in degradation products at 3 months"* (see section Results on page 1). In section 5 on page 5 of the Kapsi declaration Mr. Kapsi followed that the *"experimental data described herein demonstrates that the formulation described as Example 6 in publication WO 03*/*098992 A2 leads to a tablet formulation with a four-fold higher level of degradation products at 3 months and in my experience leads to an unacceptable tablet formulation".*

In order to avoid unacceptable degradation of eltrombopag olamine, WO 2008/136843 A1 teaches to use diluents substantially free of reducing sugars, in particular using mannitol. The avoidance of reducing sugars as pharmaceutical excipient in an eltrombopag olamine pharmaceutical tablet is continued in WO 2012/121957 A1 and WO 2015/0121957 A2.

It would, however, be desirable to provide an alternative solution for a stable eltrombopag olamine pharmaceutical tablet composition, wherein the degradation level of eltrombopag olamine is negligible and the pharmaceutical tablet composition fulfills the regulatory stability criteria for pharmaceutical tablets. Furthermore, it would be desirable that the pharmaceutical tablet composition fulfills the regulatory dissolution criteria for pharmaceutical tablets. Furthermore, it would be desirable that the pharmaceutical tablet composition is more economic in costs per unit and/or allows tuning one or more pharmaceutical technological aspects during the production process.

### Brief description of the invention:

One or more problems of the present invention is/are solved by the subjects of the independent claims. Advantages (preferred embodiments) are set out in the detailed description hereinafter as well as in the dependent claims.

Accordingly, a first aspect of the present invention relates to a pharmaceutical tablet composition comprising eltrombopag olamine as active ingredient and one or more pharmaceutically acceptable excipients including one or more reducing sugars, characterized in that the composition comprises or consists of
a) eltrombopag olamine in a therapeutically effective amount,
b) lactose as reducing sugar, and
c) povidone as polymeric binder agent,
with the proviso that in case the pharmaceutical tablet composition is obtained by granulation compression exhibiting an intragranular composition and an extragranular composition, the eltrombopag olamine, the reducing sugar and the polymeric binder agent are present in the same composition.

A second aspect of the present invention relates to an inventive pharmaceutical tablet composition for use in the treatment or prophylaxis of immune (idiopathic) thrombocytopenic purpura (ITP), thrombocytopenia and/or acquired severe aplastic anaemia (SAA).

A third aspect of the present invention relates to a process of production of an inventive pharmaceutical tablet comprising or consisting of
a) eltrombopag olamine in a therapeutically effective amount,
b) lactose as reducing sugar, and
c) povidone as polymeric binder agent and
d) optionally one or more further pharmaceutically acceptable excipients,
characterized in that the pharmaceutical tablet composition is either obtained by granulation compression or by direct compression,
with the proviso that in case the pharmaceutical tablet composition is obtained by granulation compression exhibiting an intragranular composition and an extragranular composition, the eltrombopag olamine, the one or more reducing sugars and the one or more binder agents are present in the same composition.

A fourth aspect of the present invention relates to a pharmaceutical tablet composition comprising eltrombopag olamine as active ingredient and one or more pharmaceutically acceptable excipients including one or more reducing sugars, characterized in that the composition comprising or consisting of
a) eltrombopag olamine in a therapeutically effective amount,
b) lactose as reducing sugar, and
c) povidone as polymeric binder agent,
is obtainable by the inventive production process,
with the proviso that in case the pharmaceutical tablet composition is obtained by granulation compression exhibiting an intragranular composition and an extragranular composition, the eltrombopag olamine, the one or more reducing sugars and the one or more polymeric binder agents are present in the same composition.

The inventive aspects of the present invention as disclosed hereinbefore can comprise - in case it is reasonable for a person skilled in the art - any possible combination of the preferred inventive embodiments as set out in the dependent claims or disclosed in the following detailed description.

### Detailed description of the invention:

It has surprisingly been found by the present inventors, that despite the fact that eltrombopag olamine and lactose as reducing sugar are present in the same pharmaceutical tablet composition the inventive pharmaceutical tablet composition containing povidone as a polymeric binder agent is not subjected to a substantial degradation of the active ingredient eltrombopag olamine in view of Maillard reactions with the reducing sugar and, thus, fulfills the regulatory stability requirements for commercializing the pharmaceutical eltrombopag olamine tablet (cf. Part E, table 1 of the Example section below).

In the context of the present invention the term "binder" is defined as an agent used to increase the cohesion of the powdery particles or granules during the compression, in order to obtain pharmaceutical forms with a defined hardness, or to act as processing aid during the granulation process. The binder may be present in the pharmaceutical composition in the form of a single constituent / ingredient or in the form of a mixture of constituents / ingredients. In the context of the present invention the binder agents are classified into 3 classes: 1) "natural binder", 2) "semisynthetic polymeric binder", and 3) "synthetic polymeric binder".

Due to their natural source the natural binder agents generally exhibit an inherent variability relating to the natural polymers of different batches, which sometimes gives rise to problems in production. To reduce potential processing problems, (natural) binder agents may also be further characterized, e.g., by their viscous properties. In comparison to the natural binder agents, the variability between batches of the semisynthetic and/or synthetic binder agents from the same supplier is reduced due to the adjustment by chemical derivatization or a total chemical synthesis. For almost each polymeric binder agent type, several viscosity grades are generally available.

In the context of the present invention the expression "natural binder" refers to a natural polymer binder or a salt thereof, preferably selected from the group consisting of starch, processed starch or starch salt, e.g., corn starch, potato starch, pregelatinized starch, sodium starch; alginic acid or salts thereof, e.g. sodium alginate; gelatin; Guar gum; gum Arabic; Candelilla wax; Carnauba wax.

It is disclosed herewith that the expression "semisynthetic polymeric binder" refers to a chemically modified natural polymer binder, usually a derivative of cellulose or starch, preferably selected from the group consisting of hydroxypropyl cellulose (HPC, hyprollose), hydroxypropyl methyl cellulose (HPMC, hypromellose), sodium carboxymethyl cellulose, and hydroxypropyl starch.

It ist disclosed herewith that the expression "synthetic polymeric binder" refers to a fully chemically synthesized, non-natural polymer or co-polymer binder agent, preferably selected from the group consisting of povidone, copovidone, polyvinyl alcohol, polyethylene glycol (PEG), polyvinyl caprolactam-polyvinyl acetate-polyethylene glycol graft copolymer, and polyethylene glycol-polyvinyl alcohol graft copolymer (PEG-PVA).

In the context of the present invention the expression "polymeric binder" / "polymer binder" refers to povidone.

In the context of the present invention, the expression "the eltrombopag olamine, the one or more reducing sugars and the one or more polymeric binder agents are present in the same composition" means that i) in case the inventive pharmaceutical tablet is obtained by direct compression the ingredients a) to c) are respectively present in the mixture of ingredients to be compressed to tablet cores and ii) in case the inventive pharmaceutical tablet composition is obtained by granulation compression exhibiting a granular composition (forming the intragranular composition in the tablet core) and a non-granular composition (forming the extragranular composition in the tablet core), the ingredients a) to c) are present either in the intragranular composition or in the extragranular composition or the ingredients a) to c) are present in both the intragranular and the extragranular composition respectively. Notwithstanding this definition, the ingredient b), i.e. lactose as reducing sugar, and/or ingredient c), i.e. povidone as polymeric binder agent may additionally be present in the intragranular or extragranular composition, which does not comprise eltrombopag olamine.

The finding of the present inventors is moreover surprising, as it has been disclosed in the so called Kapsi declaration that pharmaceutical compositions comprising eltrombopag olamine and lactose in the same composition are subject to severe degradation and, thus, do not fulfill the stability requirements for commercializing the respective pharmaceutical eltrombopag olamine tablet. The originator accordingly exchanged the lactose excipient by a non-reducing sugar, namely mannitol to fulfill the stability requirements.

Furthermore, the experimental data provided in table 2 in Part E) of the Example section shows that 75 wt.% or more, preferably more than 80 wt.%, more preferably more than 90 wt.% of eltrombopag olamine in the inventive pharmaceutical tablet composition is dissolved within 45 minutes in accordance with the regulatory standard test. In contrast thereto, a comparative tablet composition not comprising the polymeric binder in the same composition (cf. comparative composition C1 in the Example section) does not fulfil the dissolution requirements of at least 75 wt.% dissolution of the active ingredient after 45 minutes.

Both effects, namely reduction of degradation and increase of dissolution are presently believed to relate to the use of the claimed selection of povidon as polymeric binder, which is believed to not only function as binder agent, but also act as solubilizer agent and as a stabilizer agent. Thus, by use of the inventive teaching, namely use of povidone as polymeric binder agent, it is possible to maintain the use of a more cost effective lactose as reducing sugar as diluent constituent in the tablet formulation of eltrombopag olamine and at the same time achieve a suitable degradation and dissolution profile for the inventive pharmaceutical tablet composition.

In case the inventive pharmaceutical composition is produced by granulation compression technique (cf. Parts A and B of the Example section below), the inventive composition exhibits an intragranular composition as well as an extragranular composition.

In the context of the present invention the term "intragranular composition" / "intragranular phase" refers to tablet constituents / ingredients which are wet granulated and thus forming granules, which then form the intragranular composition / phase in the inventive pharmaceutical tablet composition. In case the intragranular tablet constituents comprise a therapeutically effective amount of eltrombopag olamine, then the intragranular composition must also comprise ingredient b), i.e. lactose, and ingredient c), i.e. povidone.

The "extragranular composition" / "extragranular phase" comprises one or more tablet constituents / ingredients, which are admixed with the granules of the intragranular composition. This mixture is subsequently compressed to form the inventive pharmaceutical tablet composition (tablet core). In case the extragranular composition comprises or consists of a therapeutically effective amount of eltrombopag olamine, then the extragranular composition must also comprise lactose as reducing sugar and povidone as polymeric binder agent.

Alternatively, the inventive pharmaceutical tablet composition may be obtained by direct (dry) compression. Direct compression provides less complex and shorter production process for producing tablets. Eltrombopag olamine as a moisture sensitive active ingredient is particularly suitable for direct compression production processes, as the risk of a Maillard reaction between eltrombopag olamine and the one or more reducing sugars is furthermore reduced.

In the context with all inventive aspects of the present invention, the inventive pharmaceutical tablet composition comprises eltrombopag olamine as the active ingredient in a therapeutically effective amount and optionally one or more further active ingredients. In the context of all aspects and preferred embodiments of the present invention, the term "eltrombopag olamine" refers to an amorphous form or one or more polymorphic crystalline forms of eltrombopag olamine, wherein eltrombopag olamine has been defined hereinbefore as eltrombopag bisethanolamine. The amorphous as well as polymorphic forms I, II, and III of eltrombopag olamine have been disclosed, e.g., in WO 2010/114943 A1. More preferably, eltrombopag olamine in particular polymorphic form I of eltrombopag olamine (cf. figure 28 of WO 2010/114943 A1 shows polymorphic form I of eltrombopag bisethanolamine) is used in accordance with all aspects and preferred embodiments of the present invention. Furthermore, the eltrombopag olamine is generally used exhibiting suitable particle sizes for formulating the inventive pharmaceutical tablet composition, wherein preferably the particle size distribution by volume (PSD) is 30 µm or less for 90 Vol. % of the particles (D 0.9), 8 µm or less for 50 Vol. % of the particles (D 0.5), and/or 2 µm or less for 10 Vol. % of the particles (D 0.1). The particle size distribution by volume may be determined by use of the Malvern technique (by volume). In preferred embodiments of all aspects of the present invention, the active ingredient eltrombopag olamine is used per tablet unit in an amount of 16.05 mg, 32.1 mg, 64.2 mg, 96.3 mg or 128.4 mg eltrombopag olamine (respectively corresponding to 12.5 mg, 25 mg, 50 mg, 75 mg or 100 mg eltrombopag free acid).

In view of eltrombopag olamine as active ingredient, the inventive pharmaceutical tablet composition is effective for use in the treatment or prophylaxis of immune (idiopathic) thrombocytopenic purpura (ITP), thrombocytopenia and/or acquired severe aplastic anaemia (SAA).

Preferably the inventive pharmaceutical tablet composition is indicated for adult chronic immune (idiopathic) thrombocytopenic purpura (ITP) in splenectomised patients who are refractory to other treatments (e.g. corticosteroids, immunoglobulins). The inventive pharmaceutical tablet composition may also be considered as second line treatment for adult non-splenectomised patients where surgery is contraindicated.

The inventive pharmaceutical tablet composition is preferably furthermore indicated in adult patients with chronic hepatitis C virus (HCV) infection for the treatment of thrombocytopenia, where the degree of thrombocytopenia is the main factor preventing the initiation or limiting the ability to maintain optimal interferon-based therapy.

The inventive pharmaceutical tablet composition is preferably furthermore indicated in adult patients with acquired severe aplastic anaemia (SAA) who were either refractory to prior immunosuppressive therapy or heavily pretreated and are unsuitable for haematopoietic stem cell transplantation.

According to all aspects of the present invention lactose is used as reducing sugar. More preferably the lactose is selected from the group consisting of lactose monohydrate, anhydrous lactose, spray dried lactose and co-processed lactose. Examples of co-processed lactose are Ludipress® (lactose monohydrate 93 wt.%, 3.5 wt.% povidone (Kollidon®30) and 3.5 wt.% crospovidone (Kollidon® CL); Cellactose® (75% lactose and 25% microcrystalline cellulose MCC); Starlac® (85% lactose and 15% starch) and Combilac® (70% lactose, 20% MCC and 10% corn starch).

The use of lactose as diluent in the tablet composition is advantageous, as lactose is in particular cheaper than other excipients and thus allows a more economic production of pharmaceutical eltrombopag olamine tablet compositions. Furthermore the use of lactose is advantageous, as a variety of commercially available grades (lactose monohydrate, anhydrous lactose and spraydried lactose) exists, which enables the skilled person to choose the best fitting grade for the present situation. Accordingly, the skilled person is enabled with respect to all aspects of the present invention to select the best fitting lactose excipient based on the required characteristics for tablet compression. For example, in case the lactose excipient shall also facilitate non-sticking at the compression facilities, lactose comprising a comparatively large particle size will be selected. In case lactose monohydrate is used, fine grades are usually used for the granulation compression process, as they permit better mixing with other excipients.

In accordance with the present invention, the reducing sugar content in the inventive pharmaceutical tablet composition is preferably greater than 5 wt.%, preferably at least 9 wt.%, more preferably in the range of 15 to 75 wt.% alternatively 30 to 70 wt.% respectively based on the total weight of the pharmaceutical tablet composition

According to an alternative embodiment of the present invention, the inventive pharmaceutical eltrombopag olamine tablet is made using at least 31 to 40 wt. % or 53 to 62 wt. % lactose, e.g. lactose monohydrate, respectively based on the total weight of the tablet. According to a preferred embodiment the inventive pharmaceutical eltrombopag tablet, the relative content of lactose in comparison to the total weight of the tablet increases with reducing the relative content of the active ingredient eltrombopag olamine.

According to the inventive pharmaceutical tablet composition, the composition also comprises or consists of povidone (non-crosslinked polyvinyl pyrrolidone, also called PVP, e.g. commercially available as Plasdone K29/32 or Kollidon® 30) as polymeric binder agent. In case co-processed lactose comprising one or more polymeric binder agents are used in the composition, then a separate amount of polymeric binder agent in the composition may be reduced (see also below paragraph). In case of granulation compression tableting, the additional use of one or more polymeric binder agents in the intragranular composition and optionally also in the extragranular composition is preferred, as the respective inventive tablet composition shows an additionally reduced degradation of eltrombopag olamine.

According to an alternative or cumulative preferred embodiment of the present invention, the use of co-processed lactose is preferred, as it already comprises a mixture of lactose and one or more further excipients. The one or more further excipients in the co-processed lactose are preferably selected from microcrystalline cellulose (MCC), starch, povidone, and crospovidone (crosslinked polyvinyl pyrrolidone, so called PVPP or polyvinyl polypyrrolidone, e.g. commercially available as Polyplasdone XL or Kollidon® CL). Commercially available co-processed lactose is available as, e.g., Ludipress® which comprises of lactose, povidone (Kollidon® 30) and crospovidone (Kollidon® CL), Cellactose® (lactose with MCC); Starlac® (lactose with starch) and Combilac® (lactose with MCC and starch). In case the further excipients of the co-processed lactose is also present as separate excipient ingredient in the granular phase not comprising the eltrombopag olamine, then the amount of the separate excipient ingredient in this other granular phase may be reduced.

According to an alternative or cumulative preferred embodiment of all aspects of the present invention, the inventive pharmaceutical tablet composition may further comprise suitable natural binder agents, preferably selected from the group consisting of starch processed starch or starch salts, e.g., corn starch, potato starch, pregelatinized starch, sodium starch; alginic acid or salts thereof, e.g. sodium alginate; gelatin, Guar gum; gum Arabic; Candelilla wax; and Carnauba wax.

Starch and pregelatinized starch (PGS) are multifunctional excipients. They can be considered as natural binder agents, but they can be used also as natural diluent agents, and furthermore also have disintegrant-like properties. A disintegrant is typically an agent used in the preparation of solid pharmaceutical formulations which causes them to disintegrate and release their medicinal substances on contact with moisture. However, the disintegrant may also be considered as a diluent and/or binder, depending on the properties of the particular excipient employed as a disintegrant. A binder is typically a substance used to create a desired consistency in a formulation, whereby a diluent is typically considered as a bulking agent to increase the mass of the formulation. Accordingly, the disintegrant exhibits properties of disintegration upon contact with aqueous environments, and is preferably a non-soluble disintegrant, but may also show properties associated with a diluent or binder. PGS is a starch that has been chemically and/or mechanically processed to rupture all or part of the starch granules. This typically renders the starch flowable and directly compressible (Handbook of Pharmaceutical Excipients (Ed: Rowe)). Partially pregelatinized starch is commercially available. In some embodiments, pregelatinized starch contains 5% of free amylose, 1 5% of free amylopectin, and 80% unmodified starch. Pregelatinized starch is typically obtained from maize (corn), potato, or rice starch.

According to an alternative or cumulative preferred embodiment of all aspects of the present invention, the inventive pharmaceutical tablet composition may further comprise suitable further diluent agents (in addition to the one or more reducing sugars), preferably one or more further diluent agents are selected from the group consisting of erythritol, isomalt, maltitol, xylitol, microcrystalline cellulose, powdered cellulose, pregelatinized starch, starch, lactitol, mannitol, sorbitol, maltodextrin more preferably one, two or more further diluent agents are selected from erythritol, isomalt, maltitol, xylitol, and microcrystalline cellulose, even more preferably the further diluent agents are selected from microcrystalline cellulose and one, two or more selected from erythritol, isomalt, maltitol and xylitol, preferably xylitol. According to an alternative or cumulative preferred embodiment of all aspects of the present invention it may be preferred that the composition comprising the eltrombopag olamine and lactose as the reducing sugar comprises at least one further diluent agent as set out above, preferably comprising microcrystalline cellulose. Such a combination is preferred as microcrystalline cellulose as insoluble diluent agent acts as counterpart of the reducing sugar representing a soluble diluent, thereby facilitating the tuning of the disintegration time of the inventive pharmaceutical tablet composition.

According to an alternative or cumulative preferred embodiment of all aspects of the present invention, the inventive pharmaceutical tablet composition - in case of granulation compression preferably the extragranular composition - may further comprise suitable one or more suitable disintegrant agents, preferably one or more disintegrant agents are selected from the group consisting of low-substituted hydroxypropyl cellulose (L-HPC), sodium starch glycolate, crospovidone, and croscarmellose sodium, more preferably one or two selected from sodium starch glycolate and crospovidone. In case, however, the inventive tablet composition is obtained by granular compression, the composition comprises one or more disintegrant agents in both the intragranular and extragranular composition, in particular in case a higher amount of eltrombopag olamine is used in the intragranular phase and/or an insoluble diluent can be used in the intragranular phase in addition to eltrombopag olamine and the reducing sugar. In this case the same or different disintegrant agent may be used both in the intragranular and extragranular composition, wherein the weight ratios of disintegrant in intra- and extragranular phase is preferably in the range of 1:1 to 1:5, in particular 1:3 or 1:4 or 1:4.6.

As an example, the intragranular phase may comprise no disintegrant agent and the extragranular phase may comprise low-substituted hydroxypropyl cellulose, sodium starch glycolate, crospovidone or croscarmellose sodium or a combination of sodium starch glycolate and crospovidone or sodium starch glycolate and croscarmellose sodium or crospovidone and croscarmellose sodium, more preferably sodium starch glycolate as disintegrant agent(s), wherein the disintegrant agent in total in the extragranular phase is in particular present in a range of 5 to 10 wt.%, preferably 8 wt.% based on the total weight of the inventive tablet composition.

As an alternative example, the intragranular phase may comprise low-substituted hydroxypropyl cellulose, sodium starch glycolate, crospovidone or croscarmellose sodium or a combination of sodium starch glycolate and crospovidone or sodium starch glycolate and croscarmellose sodium or crospovidone and croscarmellose sodium, more preferably sodium starch glycolate as disintegrant agent(s), wherein the disintegrant agent in total in the intragranular phase is in particular present in a weight range of 1 to 5 wt.%, preferably 2 wt.% based on the total weight of the inventive tablet composition, while the extragranular phase may comprise low-substituted hydroxypropyl cellulose, sodium starch glycolate, crospovidone or croscarmellose sodium, or a combination of sodium starch glycolate and crospovidone or sodium starch glycolate and croscarmellose sodium or crospovidone and croscarmellose sodium, wherein the disintegrant agent in total in the extragranular phase is in particular present in a range of 5 to 10 wt.%, preferably 8 wt.% based on the total weight of the inventive tablet composition.

According to an alternative or cumulative preferred embodiment of all aspects of the present invention, the inventive pharmaceutical composition - in case of granulation compression preferably the extragranular composition - may further comprise one or more suitable lubricant agents, preferably selected from the group consisting of magnesium stearate, stearic acid, and sodium stearyl fumarate, more preferably magnesium stearate.

According to a further alternative or cumulative preferred embodiment of all aspects of the present invention the pharmaceutical tablet composition may further be film coated with a coating agent, preferably comprising one or more pharmaceutically acceptable polymers, optionally one or more pharmaceutically acceptable plasticizers, and optionally one or more pharmaceutically acceptable pigments. The one or more polymer agents of the pharmaceutically acceptable coating agent are preferably selected from one or more of the group consisting of hydroxypropyl cellulose, hydroxypropylmethyl cellulose, ethyl cellulose, polyvinyl alcohol, polyethylene glycol, polyethylene glycol-polyvinyl alcohol graft copolymer, and polysorbate. The one or more pigments are preferably selected from the group of titanium dioxide, aluminium lakes, and iron oxides, preferably iron oxide yellow, iron oxide red, and iron oxide black.

According to an alternative or cumulative preferred embodiment of all aspects of the present invention the coating agent may comprise or consist of
- hypromellose, macrogol 400, polysorbate 80 and titanium dioxide (commercially available as Opadry® White), preferably for inventive pharmaceutical tablet compositions comprising 16.05 mg or 32.1 mg eltrombopag olamine, or
- hypromellose, macrogol 400, titanium dioxide, iron oxide yellow and iron oxide red (commercially available as Brown), preferably for inventive pharmaceutical tablet compositions comprising 64.2 mg eltrombopag olamine, or
- hypromellose, macrogol 400, titanium dioxide, iron oxide red and iron oxide black (commercially available as Brown), preferably for inventive pharmaceutical tablet compositions comprising 96.3 mg eltrombopag olamine.

According to alternative or cumulative preferred embodiments of all aspects of the present invention,
i. the total content of binder agent(s) is at least 0.5 wt.% or more, more preferably is in the range of 1 to 7 wt.%, more preferably 2 to 5 wt.% and/or
ii. the total content of diluent agent(s) including the reducing sugar amount is at least 5 wt.% or more, preferably is present in the range of 10 to 86 wt.%, alternatively in the range of 40 to 57 wt.%, alternatively in the range of 58 to 86 wt.%, alternatively in the range of 58 wt.% to 61 wt.%, alternatively in the range of 79 wt.% to 82 wt.% and/or
iii. the total content of the disintegrant agent(s) is at least 3 wt.% or more, preferably is in the range of 4 to 15 wt.%, alternatively is in the range of 6 to 10 wt.% or alternatively is in the range of 3 to 5 wt.% and/or
iv. the total content of the lubricant agent(s) is at least 0.5 wt.% or more, preferably is present in the range of 0.5 to 5 wt.%, preferably 0.8 to 3 wt.%, more preferably 0.9 to 1.5 wt.% and more preferably is 1 wt.% and/or
v. the total content of the coating agent(s) is at least 2 wt.%, preferably in the range of 3 to 7 wt.%, more preferably 4 to 5 wt.%,
   respectively based on the total weight of the pharmaceutical tablet composition.

According to one embodiment of the present invention where the inventive tablet composition is obtained by granulation compression and comprises 95,703 mg eltrombopag olamine (corresponding to 75 mg eltrombopag free acid) in the intragranular composition, the intragranular composition comprises in addition to eltrombopag olamine lactose, preferably lactose monohydrate in the range of 38 to 55 wt.%, povidone, preferably in the range of 2 to 3 wt.%, and optionally one further diluent agent, preferably insoluble diluent agent, e.g. microcrystalline cellulose, more preferably in the range of 1 to 15 wt.%, even more preferably 2 to 10 wt.%. In addition the extragranular composition may comprise one or more further diluent agents, preferably microcrystalline cellulose, preferably in the range of 1 to 15 wt.%, more preferably 2 to 10 wt.% and one or more disintegrant agents, preferably in an amount of at least at least 3 wt.% or more, preferably 6 to 12 wt.%, more preferably the disintegrant agent is sodium starch glycolate or croscarmellose sodium or a mixture of sodium starch glycolate and croscarmellose sodium. Optionally the extragranular phase may additionally comprise one or more polymeric binder agents, preferably in an amount of at least 0.5 wt.% or more, preferably 2 to 4 wt.%, more preferably 1 to 2 wt.%, more preferably povidone. In addition, the extragranular composition may comprise at least one lubricant, preferably in an amount of at least 0.5 wt.% or more, preferably 0.9 to 1.2 wt.%, more preferably the lubricant is magnesium stearate. Furthermore the inventive tablet composition may be film coated comprising suitable coating agents, preferably in an amount of least 2 wt.%, preferably 3 to 5 wt.%. The respective weight amounts as set out before respectively are based on the total weight of the inventive tablet composition. According to a furthermore preferred embodiment, the total weight of the inventive tablet composition is 348 mg relating to an uncoated tablet core unit and 363 mg per film coated tablet unit.

According to an alternative embodiment of the present invention where the inventive pharmaceutical tablet composition is obtained by direct compression and comprises 15.951 mg eltrombopag olamine per tablet unit (equivalent to 12.5 mg eltrombopag free base), the tablet composition may comprise or consist lactose preferably lactose monohydrate, in the range of 31 to 62 wt.%; povidone, e.g. povidone in the range of 1 to 3 wt.%; one or more further binder agents, preferably microcrystalline cellulose, in the range of 20 to 50 wt.%; one or more disintegrant agents, a combination of two disintegrants, preferably sodium starch glycolate and crospovidone, in the range of 3 to 8 wt.%; and one or more lubricant agents, preferably magnesium stearate, in the range of 0,5 to 2 wt.%, preferably 1 wt.%. Additionally, the inventive tablet composition may be film-coated comprising suitable agents, wherein the weight of the film coating is in the range of 3 to 5 wt.%. The respective weight amounts as set out before respectively are based on the total weight of the inventive tablet composition. According to a furthermore preferred embodiment, the total weight of the inventive tablet composition is 174 mg relating to an uncoated tablet core unit and 181.5 mg per film coated tablet unit.

According to another embodiment of all aspects of the present invention as described herein before, the inventive pharmaceutical tablet composition may additionally comprise or consist of a therapeutically effective amount of one or more additional active ingredients. In case of granulation compression, the one or more additional active ingredients may be comprised in the intragranular composition or - in case of prevention of interaction between the active ingredients and eltrombopag olamine - in the opposite composition than the one comprising eltrombopag olamine. Alternatively, the one or more additional active ingredients are simultaneously or sequentially co-administered in a separate pharmaceutical formulation. In a preferred embodiment, the one or more additional active ingredients are selected from constituents which are effective in the treatment of prophylaxis of thrombocytopenia, preferably adult chronic immune (idiopathic) thrombocytopenic purpura (ITP), acquired severe aplastic anaemia (SAA) and/or chronic hepatitis C virus (HCV) infection.

Accordingly, one or more additional active ingredients are selected from the group of corticosteroids, lithium carbonate, folate, rituximab, and immunoglobuline, in particular anti-Rh (D) immunoglobuline generally used to treat thrombocytopenia, in particular chronic immune (idiopathic) thrombocytopenic purpura (ITP).

Additionally or alternatively one or more additional active ingredients are selected from the group of anti-infective agents, such as antibiotics and antifungal agents, immunosuppressive agents, such as anti-thymocyte globulin (ATG) and cyclosporine-A (CSA) and cytokine agents, such as granulocyte colony-stimulating factor (G-CSF), granulocyte-macrophage colony-stimulating factor (GM-CSF), generally used to treat acquired severe aplastic anaemia (SAA).

Furthermore, one or more additional active ingredients are selected from the group of antiviral agents, such as daclatasvir, elbasvir, grazoprevir, ledipasvir, sofosbuvir, ombitasvir, paritaprevir, ritonavir, dasabuvir, simeprevir, sofosbuvir, velpatasvir, ribavirin, peginterferon alfa-2a or peginterferon alfa-2b generally used to treat chronic hepatitis C virus (HCV) infection.

The properties or the physical and chemical stability of the inventive pharmaceutical tablet composition may be tested in conventional manner, e.g. by measurement of appearance, hardness (or resistance to crushing), disintegration time, dissolution, friability, water content, assay for eltrombopag olamine and/or its degradation products (related substances), and/or uniformity of dosage units or mass after storage at controlled storage conditions; e.g. at intermediate and/or accelerated conditions according to ICH guideline Q1A(R2) (i.e. at 25 °C / 60 % relative humidity (RH) and/or at 40 °C / 75 % RH). These tests shall be performed according to applicable pharmaceutical regulatory standards as described e.g. in ICH or EMA guidelines and/or the European Pharmacopeia (EP).

At least some of these attributes, i.e. properties or physical and chemical stability, preferably most of these attributes and most preferably all of these attributes of the inventive pharmaceutical tablet composition are stable over time and different controlled storage conditions. In a preferred embodiment the dissolution (profile) of the inventive pharmaceutical tablet composition according to the present invention, e.g. a tablet or film-coated tablet, is stable over at least 6 months when stored preferably in Alu-Alu blisters at intermediate or long-term storage conditions, i.e. 25 °C / 60 % RH or 40 °C / 75 % RH. More preferably, dissolution and further additional attributes such as, e.g., assay, related substances or uniformity of dosage units or mass are also stable after storage over at least 6 months when stored at intermediate or long-term storage conditions. The term "stable" in the context of the present invention means that a measured value falls within range of specified values determined in accordance with a respective applicable regulatory guideline, e.g. the European Pharmacopeia.

The inventive pharmaceutical tablet composition, preferably film-coated tablet, may vary in shape and be, e.g., round, oval, oblong, cylindrical, caplet shaped or any other suitable shape, preferably it is round or caplet shaped. Furthermore, the inventive pharmaceutical tablet composition comprises means for dividing the tablet, such as scores or engravings.

The inventive pharmaceutical tablet composition, preferably film-coated tablet, has a diameter ranging between 3.5 and 15 mm and most preferably between 5.5 and 7.0 mm for round tablets and the dimension of a caplet is between 9.0 x 3.0 mm and 17.0 x 7.5 mm, preferably it is 12.7 x 5.9 mm. Thickness is ranging from 2.0 to 5.0 mm, preferably between 2.5 and 4.6 mm.

The inventive pharmaceutical tablet composition according to all aspects has a suitable hardness (or resistance to crushing), preferably the film-coated tablets may have a hardness (or resistance to crushing) of at least 40 N, preferably from 50 N to 130 N, more preferably 85 N to 110 N.

Furthermore, the inventive pharmaceutical tablet composition according to all aspects has a suitable disintegration time, preferably the film-coated tablets may have a disintegration time of at least 2 minutes, preferably in the range of 2 to 6 minutes.

Furthermore inventive pharmaceutical tablet composition, preferably film-coated tablet, may be colored and/or marked so as to impart an individual appearance and to make them instantly recognizable. The use of dyes can serve to enhance the appearance as well as to identify the inventive pharmaceutical composition. Dyes suitable for use in pharmacy typically include carotenoids, iron oxides or chlorophyll. The inventive pharmaceutical tablet composition, preferably a film-coated tablet, may be marked using an imprint code.

The inventive pharmaceutical composition may be packed in any conventional packaging known in the art, for example blisters, polypropylene or polyethylene (e.g. HDPE, high density polyethylene) containers or glass bottles. Preferably the inventive tablet composition is preferably packed in a blister known in the art, e.g. sealed aluminum blister (Alu-Alu), Aluminum / Aluminum peel-push blisters or PVC/PE/PVDC/aluminum-blisters.

According to the third aspect of the present invention the inventive process of production of a pharmaceutical tablet comprising or consisting of
a) eltrombopag olamine in a therapeutically effective amount,
b) lactose as reducing sugar, and
c) povidone as polymeric binder agent, and
d) optionally one or more further pharmaceutically acceptable excipients,
characterized in that the pharmaceutical tablet composition is either obtained by granulation compression or by direct compression,
with the proviso that in case the pharmaceutical tablet composition is obtained by granulation compression exhibiting an intragranular composition and an extragranular composition, the eltrombopag olamine, the one or more reducing sugars and the one or more polymeric binder agents are present in the same composition.

The preferred embodiments disclosed with respect to the inventive pharmaceutical tablet composition also apply in combination or separately with respect to the second aspect of the present invention.

In case the inventive pharmaceutical tablet composition is obtained by direct compression, suitable processing means are used.

Preferably the provided constituents, such as eltrombopag olamine, reducing sugars, disintegrants, (polymeric) binders, diluents, disintegrants and coating agents are sifted prior to use, preferably they are sifted through a # 35 mesh (Tyler Equivalent) screen, which corresponds to a screen with an opening of 0.420 mm in diameter.

In a cumulative or alternative preferred embodiment, the sifted materials excluding the lubricant agent(s) and coating agent(s) are loaded into a suitable blender and mixed until homogeneous distribution, e.g., for 10 minutes. Subsequently, the lubricant agent is added to the mixture and further mixed until homogeneous distribution, e.g. for further 5 minutes. Subsequently, the mixture is compressed with suitable tablet compression machines using suitable tooling and parameters into inventive tablet cores as inventive pharmaceutical tablet compositions. Preferably a rotary tablet compression machine is used. Optionally, the inventive tablet cores are furthermore film-coated with suitable coating agents in a suitable coating means, preferably a coating pan. The degradation of eltrombopag olamine may thereby be further reduced. Preferably the suitable coating agent comprises one or more polymers, optionally one or more plasticizers, and optionally one or more pigments. The preferred coating agents as disclosed with respect to the inventive pharmaceutical tablet composition can also be used with respect to the second aspect of the present invention.

Alternatively, in case the inventive pharmaceutical tablet composition is obtained by granulation compression, suitable processing means are used.

Preferably the provided constituents, such as eltrombopag olamine, reducing sugars, disintegrants, (polymeric) binders, diluents, disintegrants and coating agents are sifted prior to use, preferably they are sifted through a # 35 mesh (Tyler Equivalent) screen, which corresponds to a screen with an opening of 0.420 mm in diameter.

According to a preferred embodiment the provided constituents forming the intragranular composition, e.g., eltrombopag olamine, lactose, e.g. lactose monohydrate, further diluent agent, e.g. microcrystalline cellulose and povidone are loaded into a high shear mixer granulator or an equivalent granulator and preferably mixed for 10, more preferably 5 minutes with impeller at preferably slow speed.

Subsequently, the suitable granulation fluid, preferably water or an aqueous solution of the polymeric binder agent, is added to the mixture in the high shear mixer granulator and the impeller is preferably used at low speed.

According to a preferred embodiment the obtained granules after wet granulation are subsequently dried at suitable conditions.

In a further preferred embodiment the obtained preferably dried granules are subsequently milled, preferably using a Quadro® Comil equipped with a suitable screen.

Subsequently, the (milled, dried) granules forming the intragranular phase and the sifted and admixed extragranular constituents, i.e. further diluent agent, e.g. microcrystalline cellulose, disintegrant agent, e.g., sodium starch glycolate are loaded into a blender and mixed until homogeneous distribution, preferably for 10 minutes. In case of using a lubricant, such as magnesium stearate, this constituent is preferably subsequently added to the blender and mixed preferably until homogeneous distribution, preferably for further 5 minutes.

Subsequently, the preferably lubricated blended mixture is compressed into the inventive pharmaceutical tablet composition (tablet cores) using a suitable tablet compression machine, preferably on a rotary tablet compression machine using suitable tooling and parameters.

The resulting inventive pharmaceutical tablet composition (tablet core) may furthermore be film coated in a subsequent step using one or more pharmaceutically acceptable excipients as suitable coating agent. The degradation of eltrombopag olamine may thereby be further reduced. Preferably the suitable coating agent comprises one or more polymers, optionally one or more plasticizers, and optionally one or more pigments. The preferred coating agents as disclosed with respect to the inventive pharmaceutical tablet composition can also be used with respect to the second aspect of the present invention.

As set out in the Example Section of the present application (see Part E, table 1), the amount of impurities measured for the inventive pharmaceutical tablet compositions of the first and third aspect initially after production is below detection limit (BLD) or at 0.05 % total impurities. When storing the inventive pharmaceutical tablet compositions under accelerated conditions at open exposure on a petri dish, 50°C and 75 % relative humidity, the amount of impurities is still at a very low level. Accordingly, the inventive pharmaceutical tablet compositions of the first and third aspect show a decreased degradation of eltrombopag olamine as active ingredient in comparison to the lactose composition of the prior art (see Kapsi declaration), where the composition already initially after production comprises 1.5 % total impurities. The reduced degradation of eltrombopag olamine may be due to the use of the inventive combination of the lactose with polyvinyl pyrrolidone.

According to a fourth aspect of the present invention the inventive pharmaceutical tablet composition comprising eltrombopag olamine as active ingredient and one or more pharmaceutically acceptable excipients including one or more reducing sugars, characterized in that the composition comprising or consisting of
a) eltrombopag olamine in a therapeutically effective amount,
b) lactose as reducing sugar, and
c) povidone as polymeric binder agent,
is obtainable by use of the inventive production process,
with the proviso that in case the pharmaceutical tablet composition is obtained by granulation compression exhibiting an intragranular composition and an extragranular composition, the eltrombopag olamine, the one or more reducing sugars and the one or more polymeric binder agents are present in the same composition.

According to a fifth aspect of the present disclosure the use the inventive pharmaceutical tablet composition is disclosed, wherein the pharmaceutical tablet composition is used in the manufacture of a medicament for the treatment or prophylaxis of immune (idiopathic) thrombocytopenic purpura (ITP), thrombocytopenia and/or acquired severe aplastic anaemia (SAA). According to a sixth aspect of the present disclosure the method of treatment or prophylaxis of immune (idiopathic) thrombocytopenic purpura (ITP), thrombocytopenia and/or acquired severe aplastic anaemia (SAA), comprises administering to a patient in need an inventive pharmaceutical tablet composition is disclosed.

The present invention is explained further with the aid of the following non-limiting examples, illustrating the parameters of and compositions employed within the present invention. Unless stated otherwise, all data, in particular percentages, parts and ratios are by weight.

According to the present invention the individual features of the exemplary embodiments of the inventive pharmaceutical tablet composition and the inventive production process as disclosed in the detailed description or claims of the present application can respectively be separately combined with features of the further exemplary embodiments herein below.

### Examples:

The eltrombopag olamine constituent used in the following example formulations F1 tom F5 and C1 exhibits polymorphic form I.

### Part A: General Procedure for the wet granulation compression (eltrombopag olamine and lactose intragranular)

### Stage A: (Dry Mix / Granulation)

1. Sifted Eltrombopag olamine and intragranular excipients of Stage A through #35 mesh.
2. Sifted materials of step-1 loaded into high shear mixer granulator and mixed for 5 minutes with impeller at slow speed.
3. Step-2 material was granulated with a suitable binder solution, e.g., water or an aqueous solution such as aqueous povidone solution, at impeller slow speed.
4. The granules of step-3 material were dried in a suitable equipment to get required loss on drying.
5. The dried granules of step-4 were milled using Quadro® Comil equipped with suitable screen.

### Stage B: (Blending)

6. Extragranular materials of Stage B were sifted through #35 mesh.
7. Milled granules of step 5 and sifted extragranular materials of step 6 were loaded into the blender and mixed for 10 min.

### Stage C: (Lubrication/Tablet core compression)

8. Lubrication agents, e.g., magnesium stearate sifted through #35 mesh and added to blend mixture of step 7 and lubricated for 5 minutes.
9. The blend mixture of step 8 was compressed into tablets on rotary tablet compression machine using suitable tooling and parameters.

### Stage D: Film-Coating

10. Core tablets of step 9 were coated in coating pan using corresponding suspension.

### Part B: Process of production of inventive and disclosed pharmaceutical tablet compositions (eltrombopag olamine and lactose in the same intragranular composition)

The following inventive pharmaceutical tablet compositions **F1** and **F2** are produced in accordance with the general procedure as set out in Part A above:

### Examples F1 and F2: inventive pharmaceutical film-coated tablet compositions comprising microcrystalline cellulose, sodium starch glycolate and magnesium stearate in extragranular phase and eltrombopag olamine, lactose monohydrate, microcrystalline cellulose and polyvinyl pyrrolidone (povidone) as synthetic polymeric binder in intragranular phase

| **Ingredients** | **F1 (mg/unit)** | **F2 (mg/unit)** |
|---|---|---|
| **Stage-A: (Dry Mix / Wet Granulation)** | | |
| Eltrombopag Olamine, *In-house* | 95.703 | 95.703 |
| Lactose monohydrate *Ph.Eur.* | 138.197 | 193.037 |
| Cellulose, Microcrystalline *Ph.Eur.* | 36.500 | 9.080 |
| Polyvinyl pyrrolidone, Povidone *Ph.Eur.* (Plasdone® K29/32) | 9.600 | 9.600 |
| Water, purified *Ph.Eur.* | q.s. | q.s. |

| **Stage-B (Blending)** | | |
|---|---|---|
| Cellulose, Microcrystalline *Ph.Eur.* | 36.500 | 9.080 |
| Sodium starch glycolate Type-A *Ph.Eur.* (Explotab®) | 28.000 | 28.000 |

| **Stage-C (Lubrication)** | | |
|---|---|---|
| Magnesium stearate *Ph.Eur.* | 3.500 | 3.500 |
| **Core Tablet weight (mg)** | **348.000** | **348.000** |

| **Stage-D (Film-coating)** | | |
|---|---|---|
| Opadry® Brown *In-house* | 15.000 | 15.000 |
| Water, Purified *Ph.Eur.* | q.s. | q.s. |
| **Film-coated tablet weight (mg)** | **363.000** | **363.000** |

The following pharmaceutical tablet compositions **F3** to **F8** (not according to the invention) can be produced in accordance with the general procedure as set out in Part A above:

### Examples F3 and F4: Disclosed are pharmaceutical film-coated tablet compositions comprising microcrystalline cellulose, sodium starch glycolate and magnesium stearate in extragranular phase and eltrombopag olamine, lactose monohydrate, microcrystalline cellulose and polyvinyl alcohol or macrogol as synthetic polymeric binder in intragranular phase

| **Ingredients** | **F3 (mg/unit)** | **F4 (mg/unit)** |
|---|---|---|
| **Stage-A: (Dry Mix I Wet Granulation)** | | |
| Eltrombopag Olamine, *In-house* | 95.703 | 95.703 |
| Lactose monohydrate *Ph.Eur.* | 188.037 | 188.037 |
| Cellulose, Microcrystalline *Ph.Eur.* | 9.080 | 9.080 |
| Polyvinyl alcohol *Ph.Eur.* | 14.600 | - |
| Macrogol *Ph.Eur.* | - | 14.600 |
| Water, purified *Ph.Eur.* | q.s. | q.s. |

| **Stage-B (Blending)** | | |
|---|---|---|
| Cellulose, Microcrystalline *Ph.Eur.* | 9.080 | 9.080 |
| Sodium starch glycolate Type-A *Ph.Eur.* (Explotab®) | 28.000 | 28.000 |

| **Stage-C (Lubrication)** | | |
|---|---|---|
| Magnesium stearate *Ph.Eur.* | 3.500 | 3.500 |
| **Core Tablet weight (mg)** | **348.000** | **348.000** |

| **Stage-D (Film-coating)** | | |
|---|---|---|
| Opadry® Brown *In-house* | 15.000 | 15.000 |
| Water, Purified *Ph.Eur.* | q.s. | q.s. |
| **Film-coated tablet weight (mg)** | **363.000** | **363.000** |

### Examples F5 and F6: Disclosed are pharmaceutical film-coated tablet compositions comprising microcrystalline cellulose, sodium starch glycolate and magnesium stearate in extragranular phase and eltrombopag olamine, lactose monohydrate, microcrystalline cellulose and hydroxypropyl methylcellulose or hydroxypropyl cellulose as semisynthetic polymeric binder in intragranular phase

| **Ingredients** | **F5 (mg/unit)** | **F6 (mg/unit)** |
|---|---|---|
| **Stage-A: (Dry Mix I Wet Granulation)** | | |
| Eltrombopag Olamine, *In-house* | 95.703 | 95.703 |
| Lactose monohydrate *Ph.Eur.* | 193.037 | 193.037 |
| Cellulose, Microcrystalline *Ph.Eur.* | 9.080 | 9.080 |
| Hydroxypropyl methylcellulose *Ph.Eur.* | 9.600 | - |
| Hydroxypropyl cellulose *Ph.Eur.* | - | 9.600 |
| Water, purified *Ph.Eur.* | q.s. | q.s. |

| **Stage-B (Blending)** | | |
|---|---|---|
| Cellulose, Microcrystalline Ph.Eur. | 9.080 | 9.080 |
| Sodium starch glycolate Type-A *Ph.Eur.* (Explotab®) | 28.000 | 28.000 |

| **Stage-C (Lubrication)** | | |
|---|---|---|
| Magnesium stearate *Ph.Eur.* | 3.500 | 3.500 |
| **Core Tablet weight (mg)** | **348.000** | **348.000** |

| **Stage-D (Film-coating)** | | |
|---|---|---|
| Opadry® Brown *In-house* | 15.000 | 15.000 |
| Water, Purified *Ph.Eur.* | q.s. | q.s. |
| **Film-coated tablet weight (mg)** | **363.000** | **363.000** |

### Examples F7 and F8: Disclosed are pharmaceutical film-coated tablet compositions comprising microcrystalline cellulose, sodium starch glycolate and magnesium stearate in extragranular phase and eltrombopag olamine, lactose monohydrate, microcrystalline cellulose and hydroxypropyl starch as semisynthetic polymeric binder or polyethylene glycol-polyvinyl alcohol graft copolymer (PEG-PVA) as synthetic polymeric binder in intragranular phase

| **Ingredients** | **F7 (mg/unit)** | **F8 (mg/unit)** |
|---|---|---|
| **Stage-A: (Dry Mix I Wet Granulation)** | | |
| Eltrombopag Olamine, *In-house* | 95.703 | 95.703 |
| Lactose monohydrate *Ph.Eur.* | 193.037 | 184.037 |
| Cellulose, Microcrystalline *Ph.Eur.* | 9.080 | 9.080 |
| Hydroxypropyl starch *Ph.Eur.* | 9.600 | - |
| Polyethylene glycol-polyvinyl alcohol graft copolymer (Kollicoat® IR) | - | 18.600 |
| Water, purified *Ph.Eur.* | q.s. | q.s. |

| **Stage-B (Blending)** | | |
|---|---|---|
| Cellulose, Microcrystalline *Ph.Eur.* | 9.080 | 9.080 |
| Sodium starch glycolate Type-A *Ph.Eur.* (Explotab®) | 28.000 | 28.000 |
| **Stage-C (Lubrication)** | | |
| Magnesium stearate *Ph.Eur.* | 3.500 | 3.500 |
| **Core Tablet weight (mg)** | **348.000** | **348.000** |

| **Stage-D (Film-coating)** | | |
|---|---|---|
| Opadry® Brown *In-house* | 15.000 | 15.000 |
| Water, Purified *Ph.Eur.* | q.s. | q.s. |
| **Film-coated tablet weight (mg)** | **363.000** | **363.000** |

### Part C: General Procedure for the direct compression (eltrombopag olamine and lactose in one composition)

### Stage A: (Dry Mixing / Blending)

1. Stage-A materials were sifted through #35 mesh and collected separately.
2. The sifted materials of step-1 were loaded in to the blender and mixed for 10 min.

### Stage B: (Lubrication / Direct compression of core tablets)

3. Stage-B lubrication agent sifted through #35 mesh and added to step-2 blend and lubricated for 5 minutes.
4. Compressed the step-3 blend into tablet cores on rotary tablet compression machine using suitable tooling and parameters.

### Stage C: (Film Coating)

5. Core tablets of step-4 were coated in coating pan using White suspension.

### Part D: Process of production of inventive, disclosed and comparative pharmaceutical tablet compositions (direct compression of eltrombopag olamine and lactose) Compositions F12-F14 are not according to the invention (comparative compositions)

The following inventive pharmaceutical tablet compositions **F9** to **F11** are produced in accordance with the general procedure as set out in Part C above.

### Examples F9 to F11: inventive pharmaceutical film-coated tablet compositions comprising eltrombopag olamine, Ludipress® (93 wt.% lactose monohydrate, 3.5 wt.% polyvinyl pyrrolidone (Kollidon® 30) and 3.5 wt.% crospovidone (Kollidon® CL)) or lactose monohydrate and povidone, microcrystalline cellulose, sodium starch glycolate and magnesium stearate

| **Ingredients** | **F9 (mg/unit)** | **F10 (mg/unit)** | **F11 (mg/unit)** |
|---|---|---|---|
| **Stage-A: (Blending)** | | | |
| Eltrombopag Olamine, *In-house* | 15.951 | 15.951 | 15.951 |
| Ludipress® (93% Lactose monohydrate, 3.5% Povidone, and 3.5% Crospovidone) | 60.750 | 112.850 | - |
| Lactose monohydrate *Ph.Eur.* | - | - | 107.300 |
| Cellulose, Microcrystalline *Ph.Eur.* | 88.609 | 36.509 | 36.459 |
| Sodium starch glycolate Type-A *Ph.Eur.* (Explotab®) | 6.940 | 6.940 | 6.940 |
| Polyvinyl pyrrolidone, Povidone *Ph.Eur.* (Plasdone® K29/32) | - | - | 5.600 |

| **Stage-B (Lubrication)** | | | |
|---|---|---|---|
| Magnesium stearate *Ph.Eur.* | 1.750 | 1.750 | 1.750 |
| **Core Tablet weight (mg)** | **174.000** | **174.000** | **174.000** |

| **Stage-C (Film-coating)** | | | |
|---|---|---|---|
| Opadry® White *In-house* | 7.500 | 7.500 | 7.500 |
| Water, Purified *Ph.Eur.* | q.s. | q.s. | q.s. |
| **Film-coated tablet weight (mg)** | **181.500** | **181.500** | **181.500** |

The following disclosed pharmaceutical tablet compositions **F12** to **F11** can be produced in accordance with the general procedure as set out in Part C above.

### Examples F12 to F14: Disclosed are pharmaceutical film-coated tablet compositions comprising eltrombopag olamine, lactose monohydrate, microcrystalline cellulose, sodium starch glycolate, copovidone or polyvinyl caprolactam-polyvinyl acetate-polyethylene glycol graft copolymer as synthetic polymeric binder or carmellose sodium (Na-CMC) as semisynthetic polymeric binder and magnesium stearate

| **Ingredients** | **F12 (mg/unit)** | **F13 (mg/unit)** | **F14 (mg/unit)** |
|---|---|---|---|
| **Stage-A: (Blending)** | | | |
| Eltrombopag Olamine, *In-house* | 15.951 | 15.951 | 15.951 |
| Lactose monohydrate *Ph.Eur.* | 107.300 | 107.300 | 107.300 |
| Cellulose, Microcrystalline *Ph.Eur.* | 36.459 | 36.459 | 36.459 |
| Sodium starch glycolate Type-A *Ph.Eur.* (Explotab®) | 6.940 | 6.940 | 6.940 |
| Copovidone *Ph. Eur.* (Kollidon® VA64 Fine) | 5.600 | - | - |
| Polyvinyl caprolactam-polyvinyl acetate-polyethylene glycol graft copolymer (So-luplus®) | - | 5.600 | |
| Carmellose sodium *Ph.Eur.* | - | - | 5.600 |

| **Stage-B (Lubrication)** | | | |
|---|---|---|---|
| Magnesium stearate *Ph.Eur.* | 1.750 | 1.750 | 1.750 |
| **Core Tablet weight (mg)** | **174.000** | **174.000** | **174.000** |

| **Stage-C (Film-coating)** | | | |
|---|---|---|---|
| Opadry® White *In-house* | 7.500 | 7.500 | 7.500 |
| Water, Purified *Ph.Eur.* | q.s. | q.s. | q.s. |
| **Film-coated tablet weight (mg)** | **181.500** | **181.500** | **181.500** |

The following comparative example formulation **C1** is produced in accordance with the general procedure as set out in Part C above.

### Comparative Example C1: comparative pharmaceutical film-coated tablet composition comprising eltrombopag olamine, lactose monohydrate, microcrystalline cellulose, sodium starch glycolate, and magnesium stearate

| **Ingredients** | **C1 (mg/unit)** |
|---|---|
| **Stage-A: (Blending)** | |
| Eltrombopag Olamine, *In-house* | 15.951 |
| Lactose monohydrate *Ph.Eur.* | 60.75 |
| Cellulose, Microcrystalline *Ph.Eur.* | 88.609 |
| Sodium starch glycolate Type-A *Ph.Eur.* (Explotab®) | 6.940 |
| **Stage-B (Lubrication)** | |
| Magnesium stearate *Ph.Eur.* | 1.750 |
| **Core Tablet weight (mg)** | **174.000** |
| **Stage-C (Film-coating)** | |
| Opadry® White *In-house* | 7.500 |
| Water, Purified *Ph.Eur.* | q.s. |
| **Film-coated tablet weight (mg)** | **181.500** |

### Part E: Stability and dissolution of pharmaceutical tablet compositions

**Table 1. ASAP Stability data of Coated Tablets, Open Exposure:**

| **Composition** | **Condition/ Stage** | **% Unspecified impurity** | **% Total impurities** |
|---|---|---|---|
| **F1** | Initial | BDL | BDL |
| | 50°C/75%RH 14 days | 0.34 | 0.83 |
| **F2** | Initial | BDL | BDL |
| | 50°C/75%RH 14 days | 0.14 | 0.42 |
| **F9** | Initial | 0.05 | 0.05 |
| | 50°C/75%RH 14 days | 0.23 | 0.30 |
| **F10** | Initial | 0.05 | 0.05 |
| | 50°C/75%RH 14 days | 0.29 | 0.60 |
| **F11** | Initial | 0.05 | 0.05 |
| | 50°C/75%RH 14 days | 0.21 | 0.48 |

An Accelerated Stability Assessment Program (ASAP) for up to 2-3 weeks under open exposure, according to Waterman 2011 (Waterman KC, The application of the Accelerated Stability Assessment Program (ASAP) to quality by design (QbD) for drug product stability, AAPS PharmSciTech, Vol. 12, No. 3, September 2011*)* is usually applied for a quick estimation of chemical stability (cf. Table 1).

According to the present experimental set up, the storage was conducted at 50 °C, 75 % relative humidity for 14 days at open exposure (ASAP data).

The amount of impurities measured for the inventive pharmaceutical tablet compositions **F1, F2,** and **F9** to **F11** initially after production is respectively below detection limit or at 0.05 % unspecified / total impurities.

The amount of impurities measured for the inventive pharmaceutical tablet compositions **F1, F2,** and **F9** to **F11** after storage (ASAP data) show that the inventive tablet composition has a suitable low degradation profile.

**Table 2. Dissolution testing:**

| **Composition** | **Cumulative wt.% eltrombopag olamine after 45 minutes** |
|---|---|
| **F1** | **80** |
| **F2** | **89** |
| **F9** | **81** |
| **F10** | **95** |
| **F11** | **92** |
| **C1** | **72** |

Dissolution testing according to table 2 was performed using USP Apparatus II, 50 rpm, 900 ml of phosphate buffer pH 6.8 containing 0.5% Tween 80 as recommended for Eltrombopag Olamine in the FDA Dissolution Methods Database on the FDA webpage. Based on the Ph. Eur. (5.17.1) recommendation for immediate-release dosage forms, the specification is set as at least 75% of the active substance is dissolved within 45 minutes.

The experimental data of table 2 shows that 75 wt.% or more, preferably more than 80 wt.%, more preferably more than 90 wt.% of eltrombopag olamine of the inventive pharmaceutical tablet compositions **F1, F2,** and **F9** to **F11** is dissolved within 45 minutes in accordance with the regulatory standard test.

In comparison thereto, the comparative composition **C1** does not fulfil the regulatory standard test for dissolution, as only 72 wt.% of eltrombopag olamine is dissolved within 45 minutes.

## Claims

1. Pharmaceutical tablet composition comprising eltrombopag olamine as active ingredient and one or more pharmaceutically acceptable excipients including one or more reducing sugars, **characterized in that** the composition comprises or consists of
a) eltrombopag olamine in a therapeutically effective amount,
b) lactose as reducing sugar, and
c) povidone as polymeric binder agent,
with the proviso that in case the pharmaceutical tablet composition is obtained by granulation compression exhibiting an intragranular composition and an extragranular composition, the eltrombopag olamine, the reducing sugar and the polymeric binder agent are present in the same composition.

2. Pharmaceutical tablet composition according to claim 1, wherein the reducing sugar in the extragranular composition comprises or consists of lactose selected from the group consisting of lactose monohydrate, anhydrous lactose, spray dried lactose and co-processed lactose.

3. Pharmaceutical tablet composition according to claim 1 or 2, wherein the reducing sugar content is greater than 5 wt.%, preferably at least 9 wt.%, more preferably in the range of 15 to 75 wt.% alternatively 30 to 70 wt.% respectively based on the total weight of the pharmaceutical tablet composition.

4. Pharmaceutical tablet composition according to any one of claims 1 to 3, wherein the composition comprises one or more further pharmaceutical acceptable excipient selected from
i. one or more suitable further natural binder agents, preferably selected from the group consisting of starch or starch derivatives, e.g., corn starch, potato starch, pregelatinized starch, sodium starch; alginic acid or salts thereof, e.g. sodium alginate; gelatin; Guar gum; gum Arabic; Candelilla wax; and Carnauba wax and/or
ii. one or more suitable further diluent agents, preferably one or more further diluent agents are selected from the group consisting of erythritol, isomalt, maltitol, xylitol, microcrystalline cellulose, powdered cellulose, pregelatinized starch, starch, lactitol, mannitol, sorbitol, maltodextrin more preferably one, two or more further diluent agents are selected from erythritol, isomalt, maltitol, xylitol, and microcrystalline cellulose, even more preferably the further diluent agents are selected from microcrystalline cellulose and one, two or more selected from erythritol, isomalt, maltitol and xylitol, preferably xylitol, and/or
iii. one or more suitable disintegrant agents, preferably one or more disintegrant agents are selected from the group consisting of low-substituted hydroxypropyl cellulose, sodium starch glycolate, crospovidone, and croscarmellose sodium, more preferably one or two selected from sodium starch glycolate and crospovidone, and/or
iv. one or more suitable lubricant agents, preferably selected from magnesium stearate, stearic acid, and sodium stearyl fumarate, more preferably magnesium stearate, and/or
v. one, two, three, four or more suitable coating agents, preferably comprising one or more pharmaceutically acceptable polymers, optionally one or more pharmaceutically acceptable plasticizers, and optionally one or more pharmaceutically acceptable pigments.

5. Pharmaceutical tablet composition according to any one of claims 1 to 4, wherein
i. the total content of binder agent(s) is at least 0.5 wt.% or more, more preferably is in the range of 1 to 7 wt.%, more preferably 2 to 5 wt.% and/or
ii. the total content of diluent agent(s) including the reducing sugar amount is at least 5 wt.% or more, preferably is present in the range of 10 to 86 wt.%, alternatively in the range of 40 to 57 wt.%, alternatively in the range of 58 to 86 wt.%, alternatively in the range of 58 wt.% to 61 wt.%, alternatively in the range of 79 wt.% to 82 wt.% and/or
iii. the total content of the disintegrant agent(s) is at least 3 wt.% or more, preferably is in the range of 4 to 15 wt.%, alternatively is in the range of 6 to 10 wt.% or alternatively is in the range of 3 to 5 wt.% and/or
iv. the total content of the lubricant agent(s) is at least 0.5 wt.% or more, preferably is present in the range of 0.5 to 5 wt.%, preferably 0.8 to 3 wt.%, more preferably 0.9 to 1.5 wt.% and more preferably is 1 wt.% and/or
v. the total content of the coating agent(s) is at least 2 wt.%, preferably in the range of 3 to 7 wt.%, more preferably 4 to 5 wt.%,
respectively based on the total weight of the pharmaceutical tablet composition.

6. Pharmaceutical tablet composition according to any one of the preceding claims for use in the treatment or prophylaxis of immune (idiopathic) thrombocytopenic purpura (ITP), thrombocytopenia and/or acquired severe aplastic anaemia (SAA).

7. Process of production of a pharmaceutical tablet composition comprising or consisting of
a) eltrombopag olamine in a therapeutically effective amount,
b) lactose as reducing sugar, and
c) povidone as polymeric binder agent, and
d) optionally one or more further pharmaceutically acceptable excipients,
**characterized in that** the pharmaceutical tablet composition is either obtained by granulation compression or by direct compression,
with the proviso that in case the pharmaceutical tablet composition is obtained by granulation compression exhibiting an intragranular composition and an extragranular composition, the eltrombopag olamine, the reducing sugar and the polymeric binder agent are present in the same composition.

8. Process according to claim 7, wherein the obtained pharmaceutical tablet composition is subsequently film coated with one or more pharmaceutically acceptable excipients.

9. Pharmaceutical tablet composition comprising eltrombopag olamine as active ingredient and one or more pharmaceutically acceptable excipients including one or more reducing sugars, **characterized in that** the composition comprising or consisting of
a) eltrombopag olamine in a therapeutically effective amount,
b) lactose as reducing sugar, and
c) povidone as polymeric binder agent,
is obtainable by the production process according to claim 7 or 8,
with the proviso that in case the pharmaceutical tablet composition is obtained by granulation compression exhibiting an intragranular composition and an extragranular composition, the eltrombopag olamine, the one or more reducing sugars and the one or more polymeric binder agents are present in the same composition.

## Patentansprüche

1. Pharmazeutische Tablettenzusammensetzung umfassend Eltrombopag-Olamin als Wirkstoff und einen oder mehrere pharmazeutisch akzeptable Hilfsstoffe einschließlich eines oder mehrerer reduzierender Zucker, **dadurch gekennzeichnet, dass** die Zusammensetzung umfasst oder besteht aus
a) Eltrombopag-Olamin in einer therapeutisch wirksamen Menge,
b) Lactose als reduzierender Zucker und
c) Povidon als polymeres Bindemittel,
mit der Maßgabe, dass Eltrombopag-Olamin, der reduzierende Zucker und das polymere Bindemittel in derselben Zusammensetzung vorliegen, falls die pharmazeutische Tablettenzusammensetzung durch Granulatverpressung erhalten wird, die eine intragranulare Zusammensetzung und eine extragranulare Zusammensetzung zeigt.

2. Pharmazeutische Tablettenzusammensetzung gemäß Anspruch 1, wobei der reduzierende Zucker in der extragranularen Zusammensetzung umfasst oder besteht aus Lactose, ausgewählt aus der Gruppe bestehend aus Lactose-Monohydrat, wasserfreier Lactose, sprühgetrockneter Lactose oder coprozessierter Lactose.

3. Pharmazeutische Tablettenzusammensetzung gemäß Anspruch 1 oder 2, wobei der reduzierende Zuckeranteil größer als 5 Gew.-% ist, vorzugsweise zumindest 9 Gew.-%, bevorzugter im Bereich zwischen 15 bis 75 Gew.-%, alternativ 30 bis 70% Gew.-%, jeweils bezogen auf das Gesamtgewicht der pharmazeutischen Zusammensetzung.

4. Pharmazeutische Tablettenzusammensetzung gemäß einem der Ansprüche 1 bis 3, wobei die Zusammensetzung einen oder mehrere weitere pharmazeutisch verträgliche Hilfsstoffe umfasst, ausgewählt aus
i. einem oder mehreren geeigneten weiteren natürlichen Bindemitteln, vorzugsweise ausgewählt aus der Gruppe bestehend aus Stärke oder Stärkederivaten, z.B. Maisstärke, Kartoffelstärke, vorgelatinierte Stärke, Natriumstärke; Alginsäure oder Salze davon, z.B. Natriumalginat, Gelatine, Guarkernmehl, Gummi arabicum, Candelillawachs, und Carnaubawachs und/oder
ii. einem oder mehreren geeigneten weiteren Füllstoffen, vorzugsweise einem oder mehreren weiteren Füllstoffen ausgewählt aus der Gruppe bestehend aus Erythritol, Isomalt, Maltitol, Xylitol, mikrokristalliner Cellulose, Cellulosepulver, vorgelatinierter Stärke, Stärke, Lactitol, Mannitol, Sorbitol, Maltodextrin, bevorzugter einem, zwei oder mehreren weiteren Füllstoffen ausgewählt aus Erythritol, Isomalt, Maltitol, Xylitol und mikrokristalliner Cellulose, noch bevorzugter sind die weiteren Füllstoffe ausgewählt aus mikrokristalliner Cellulose und einem, zwei oder mehreren ausgewählt aus Erythritol, Isomalt, Maltitol und Xylitol, vorzugsweise Xylitol, und/oder
iii. einem oder mehreren geeigneten Sprengmitteln, vorzugsweise einem oder mehreren Sprengmitteln ausgewählt aus der Gruppe bestehend aus niedrigsubstituierter Hydroxypropylcellulose, Natrium-Stärke-Glykolat, Crospovidon und Croscarmellose-Natrium, bevorzugter einem oder zwei ausgewählt aus Natrium-Stärke-Glykolat und Crospovidon, und /oder
iv. einem oder mehreren geeigneten Schmiermitteln, vorzugsweise ausgewählt aus Magnesiumstearat, Stearinsäure und Natriumstearylfumarat, bevorzugter Magnesiumstearat, und/oder
v. einem, zwei, drei, vier oder mehreren geeigneten Filmüberszugsmittteln, vorzugsweise umfassend ein oder mehrere pharmazeutisch akzeptable Polymere, gegebenenfalls einen oder mehrere pharmazeutisch akzeptable Weichmacher und gegebenenfalls ein oder mehrere pharmazeutisch akzeptable Pigmente.

5. Pharmazeutische Tablettenzusammensetzung gemäß einem der Ansprüche 1 bis 4, wobei
i. der Gesamtanteil des/der Bindemittel(s) zumindest 0,5 Gew.-% oder mehr beträgt, bevorzugter in dem Bereich von 1 bis 7 Gew.-% liegt, bevorzugter von 2 bis 5 Gew.-%, und/oder
ii. der Gesamtanteil des/der Füllstoffe(s) einschließlich des reduzierenden Zuckeranteils zumindest 5 Gew.-% oder mehr beträgt, vorzugsweise im Bereich von 10 bis 86 Gew.-% liegt, alternativ im Bereich von 40 bis 57 Gew.-%, alternativ im Bereich von 58 bis 86 Gew.-%, alternativ im Bereich von 58 bis 61 Gew.-%, alternativ im Bereich von 79 bis 82 Gew.-%, und/oder
iii. der Gesamtanteil des/der Sprengmittel(s) zumindest 3 Gew.-% oder mehr beträgt, vorzugsweise im Bereich von 4 bis 15 Gew.-% liegt, alternativ im Bereich von 6 bis 10 Gew.-% oder alternativ im Bereich von 3 bis 5 Gew.-%, und/oder
iv. der Gesamtanteil des/der Schmiermittel(s) zumindest 0,5 Gew.-% oder mehr beträgt, vorzugsweise im Bereich von 0,5 bis 5 Gew.-% liegt, vorzugsweise 0,8 bis 3 Gew.-%, bevorzugter 0,9 bis 1,5 Gew.-% und bevorzugter 1 Gew.-%, und/oder
v. der Gesamtanteil des/der Filmüberzugsmittel(s) zumindest 2 Gew.-% beträgt, vorzugsweise im Bereich von 3 bis 7 Gew.-% liegt, bevorzugter 4 bis 5 Gew.-%,
jeweils bezogen auf das Gesamtgewicht der pharmazeutischen Tablettenzusammensetzung.

6. Pharmazeutische Tablettenzusammensetzung gemäß einem der vorstehenden Ansprüche zur Verwendung in der Behandlung oder Prophylaxe von immun (idiopathischer) thrombozytopenischer Purpura (ITP), Thrombozytopenie und/oder erworbener schwerer aplastischer Anämie (SAA).

7. Verfahren zur Herstellung einer pharmazeutischen Tablette umfassend oder bestehend aus:
a. Eltrombopag-Olamin in einer therapeutisch wirksamen Menge,
b. Lactose als reduzierender Zucker,
c. Povidon als polymeres Bindemittel, und
d. gegebenenfalls einem oder mehreren weiteren pharmazeutisch akzeptablen Hilfsstoffen,
**dadurch gekennzeichnet, dass** die pharmazeutische Tablettenzusammensetzung entweder durch Granulatverpressung oder durch Direktverpressung erhalten wird,
mit der Maßgabe, dass Eltrombopag-Olamin, der reduzierende Zucker und das polymere Bindemittel in derselben Zusammensetzung vorliegen, falls die pharmazeutische Tablettenzusammensetzung durch Granulatverpressung erhalten wird, die eine intragranulare Zusammensetzung und eine extragranulare Zusammensetzung zeigt.

8. Verfahren gemäß Anspruch 7, wobei die erhaltene pharmazeutische Tablettenzusammensetzung anschließend mit einem oder mehreren pharmazeutisch akzeptablen Hilfsstoffen filmbeschichtet wird.

9. Pharmazeutische Tablettenzusammensetzung umfassend Eltrombopag-Olamin als Wirkstoff und einen oder mehrere pharmazeutisch akzeptable Hilfsstoffe einschließlich eines oder mehrerer reduzierender Zucker, **dadurch gekennzeichnet, dass** die Zusammensetzung umfassend oder bestehend aus:
a) Eltrombopag-Olamin in einer therapeutisch wirksamen Menge,
b) Lactose als reduzierender Zucker, und
c) Povidon als polymeres Bindemittel
nach dem Verfahren gemäß Anspruch 7 oder 8 erhältlich ist,
mit der Maßgabe, dass Eltrombopag-Olamin, der eine oder die mehreren reduzierenden Zucker und das eine oder die mehreren polymeren Bindemittel in derselben Zusammensetzung vorliegen, falls die pharmazeutische Tablettenzusammensetzung durch Granulatverpressung erhalten wird, die eine intragranulare Zusammensetzung und eine extragranulare Zusammensetzung zeigt.

## Revendications

1. Composition de comprimé pharmaceutique comprenant d'eltrombopag olamine comme ingrédient actif et un ou plusieurs excipients pharmaceutiquement acceptables comprenant un ou plusieurs sucres réducteurs, **caractérisée en ce que** la composition comprend ou consiste en
a) l'eltrombopag olamine en une quantité thérapeutiquement efficace,
b) le lactose comme sucre réducteur, et
c) la povidone comme agent liant polymère,
à condition que, dans le cas où la composition de comprimé pharmaceutique soit obtenue par compression du granulat présentant une composition intragranulaire et une composition extragranulaire, l'eltrombopag olamine, le sucre réducteur et l'agent liant polymère sont présents dans la même composition.

2. Composition de comprimé pharmaceutique selon la revendication 1, dans laquelle le sucre réducteur dans la composition extragranulaire comprend ou consiste en du lactose choisi dans le groupe consistant en du lactose monohydraté, du lactose anhydre, du lactose séché par pulvérisation et du lactose co-traité.

3. Composition de comprimé pharmaceutique selon la revendication 1 ou 2, dans laquelle le taux de sucre réducteur est supérieur à 5% en poids, de préférence à au moins 9% en poids, encore plus préférablement dans la gamme de 15 à 75% en poids, alternativement de 30 à 70% en poids, par rapport au poids total de la composition de comprimé pharmaceutique.

4. Composition de comprimé pharmaceutique selon l'une quelconque des revendications 1 à 3, dans laquelle la composition comprend un ou plusieurs autres excipients pharmaceutiquement acceptables choisis parmi
i. un ou plusieurs autres agents liants naturels appropriés, de préférence choisis dans le groupe consistant en amidon ou dérivés d'amidon, par exemple, amidon de maïs, amidon de pomme de terre, amidon prégélatinisé, amidon sodique, l'acide alginique ou ses sels, par ex. alginate de sodium, gélatine, la gomme de guar, la gomme arabique, cire de candelilla; et de la cire de carnauba, et/ou
ii. un ou plusieurs autres agents diluants appropriés, de préférence un ou plusieurs autres agents diluants sont choisis dans le groupe constitué par l'érythritol, l'isomalt, le maltitol, le xylitol, la cellulose à microcristalline, la cellulose en poudre, l'amidon prégélatinisé, l'amidon, le lactitol, le mannitol, le sorbitol, la maltodextrine, plus préférablement un, deux ou plusieurs autres agents diluants sont choisis parmi l'érythritol, l'isomalt, le maltitol, le xylitol et la cellulose microcristalline, encore plus préférablement les autres agents diluants sont choisis parmi la cellulose microcristalline et un, deux ou plus sélectionnés parmi l'érythritol , l'isomalt, le maltitol et le xylitol, de préférence le xylitol, et/ou
iii. un ou plusieurs agents désintégrants appropriés, de préférence un ou plusieurs agents désintégrants sont choisis dans le groupe constitué par l'hydroxypropylcellulose faiblement substituée, le glycolate d'amidon sodique, la crospovidone et la croscarmellose sodique, plus préférablement un ou deux choisis parmi le glycolate d'amidon sodique et la crospovidone, et/ou
iv. un ou plusieurs agents lubrifiants appropriés, de préférence choisis parmi le stéarate de magnésium, l'acide stéarique et le stéaryl fumarate de sodium, plus préférablement le stéarate de magnésium, et/ou
v. un, deux, trois, quatre ou plusieurs agents d'enrobage appropriés, comprenant de préférence un ou plusieurs polymères pharmaceutiquement acceptables, éventuellement un ou plusieurs plastifiants pharmaceutiquement acceptables, et éventuellement un ou plusieurs pigments pharmaceutiquement acceptables.

5. Composition de comprimé pharmaceutique selon l'une quelconque des revendications 1 à 4, dans laquelle
i. la teneur totale en agent(s) liant(s) est au moins de 0,5% en poids ou plus, plus préférablement dans la gamme de 1 à 7% en poids, plus préférablement de 2 à 5% en poids, et/ou
ii. la teneur totale en agent(s) diluant(s), y compris la quantité de sucre réducteur, est au moins de 5% en poids ou plus, préférablement se trouve dans la gamme de 10 à 86% en poids, alternativement dans la gamme de 40 à 57 % en poids, alternativement dans la gamme de 58 à 86% en poids, alternativement dans la gamme de 58% en poids à 61% en poids, alternativement dans la gamme de 79% en poids à 82% en poids, et/ou
iii. la teneur totale en agent(s) désintégrant(s) est au moins de 3% en poids ou plus, préférablement est dans la gamme de 4 à 15% en poids, alternativement dans la gamme de 6 à 10% en poids, alternativement dans la gamme de 3 à 5% en poids, et/ou
iv. la teneur totale en agent(s) lubrifiant(s) est au moins de 0,5% en poids ou plus, préférablement se trouve dans la gamme de 0,5 à 5% en poids, préférablement de 0,8 à 3% en poids, plus préférablement de 0,9 à 1,5% en poids et plus préférablement est de 1% en poids, et/ou
v. la teneur totale en agent(s) d'enrobage est au moins de 2% en poids, préférablement dans la gamme de 3 à 7% en poids, plus préférablement de 4 à 5% en poids,
par rapport au poids total de la composition de comprimé pharmaceutique.

6. Composition de comprimé pharmaceutique selon l'une quelconque des revendications précédentes pour utilisation pour le traitement ou la prévention de la purpura thrombopénique immunologique (idiopathique) (PTI), la thrombocytopénie et/ou l'aplasie médullaire acquise sévère (AMS).

7. Procédé de production d'une composition de comprimé pharmaceutique comprenant ou consistant en :
a. l'eltrombopag olamine en une quantité thérapeutiquement efficace,
b. le lactose comme sucre réducteur, et
c. la povidone comme agent liant polymère, et
d. éventuellement un ou plusieurs autres excipients pharmaceutiquement acceptables,
**caractérisé en ce que** la composition de comprimé pharmaceutique est obtenue soit par compression du granulat soit par compression directe,
à condition que, dans le cas où la composition de comprimé pharmaceutique soit obtenue par compression du granulat présentant une composition intragranulaire et une composition extragranulaire, l'eltrombopag olamine, le sucre réducteur et l'agent liant polymère sont présents dans la même composition.

8. Procédé selon la revendication 7, dans lequel la composition de comprimé pharmaceutique obtenue est ensuite pelliculée avec un ou plusieurs excipients pharmaceutiquement acceptables.

9. Composition de comprimé pharmaceutique comprenant d'eltrombopag olamine comme ingrédient actif et un ou plusieurs excipients pharmaceutiquement acceptables comprenant un ou plusieurs sucres réducteurs, **caractérisé en ce que** la composition comprenant ou consistant en
a) l'eltrombopag olamine en une quantité thérapeutiquement efficace,
b) le lactose comme sucre réducteur, et
c) la povidone comme agent liant polymère,
peut être obtenue par le procédé de production selon la revendication 7 ou 8,
à la condition que, dans le cas où la composition pharmaceutique en comprimés soit obtenue par compression du granulat présentant une composition intragranulaire et une composition extragranulaire, l'eltrombopag olamine, le ou les sucres réducteurs et le ou les agents liants polymériques sont présents dans la même composition .
